(19) Europäisches Patentamt
European Patent Office
Office européen des brevets

(11) Publication number: **0 559 894 A1**

(12) # EUROPEAN PATENT APPLICATION published in accordance with Art. 158(3) EPC

(21) Application number: **91917828.5**

(51) Int. Cl.$^5$: **A61K 7/06**

(22) Date of filing: **17.10.91**

(86) International application number: **PCT/JP91/01416**

(87) International publication number: **WO 92/09258 (11.06.92 92/13)**

(30) Priority: **30.11.90 JP 337857/90**

(43) Date of publication of application: **15.09.93 Bulletin 93/37**

(84) Designated Contracting States: **AT BE CH DE DK ES FR GB GR IT LI LU NL SE**

(71) Applicant: **TAISHO PHARMACEUTICAL CO. LTD**
**24-1 Takata 3-chome Toshima-ku**
**Tokyo 171(JP)**

(72) Inventor: **NAGASHIMA, Shin-ichi,**
**Sanraitodaido 202**
**1985-5, Kawarabuki Ageo-shi**
**Saitama 362(JP)**
Inventor: **MITSUYAMA, Syunpei,**
**Nishiageo-dainidanchi 1-8-306**
**77-1, Oaza-koshikiya**
**Ageo-shi Saitama 362(JP)**
Inventor: **TANAKA, Shigeo, 532-6, Mukouyama**
**Ageo-shi**
**Saitama 362(JP)**
Inventor: **NEMOTO, Masami, 1214-75, Sakata**
**Okegawa-shi**
**Saitama 363(JP)**

(74) Representative: **Weisert, Annekäte, Dipl.-Ing.**
**Dr.-Ing. et al**
**Patentanwälte Kraus Weisert & Partner**
**Thomas-Wimmer-Ring 15**
**D-80539 München (DE)**

(54) **NOVEL USE OF ALKANAMIDOAMMONIUM COMPOUND.**

(57) Object: to provide a novel type of hair growth promoter which has a remarkable action of promoting hair growth and little irritates the skin. Consitution: a hair growth promoter containing an alkanamidoammonium compound represented by general formula (I) as the active ingredient, wherein $R^1$ represents $C_7$ to $C_{25}$ alkyl; $R^2$, $R^3$ and $R^4$ may be the same or different from one another and each represents $C_1$ to $C_4$ alkyl; m represents an integer of 2 to 4; and X represents an atom or atomic group acting as an anion.

EP 0 559 894 A1

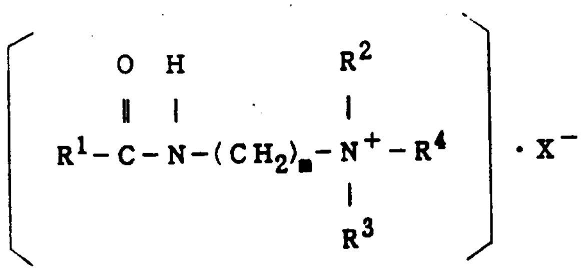

(I)

## TECHNICAL FIELD

The present invention relates to a new use of alkanamidoammonium compounds, and more particularly relates to a new use of alkanamidoanmonium compounds for promoting hair generation of mammals including humans.

## BACKGROUND ART

In order to improve alopecia in mammals, there have heretofore been used substances having the hair generation promoting effect such as, for example, calpronium chloride, estradiol, swertiamarin, glycerol pentadecanate and ginseng extract.

On the other hand, certain alkanamidoammonium compounds are known in Japanese Patent Kokai No. 61-179289, but not found to have the hair generation promoting effect.

An object of the present invention is to provide a new type of the hair generation promoting agent with an excellent hair generation promoting effect and low skin irritation.

## DISCLOSURE OF INVENTION

As a result of the various researches to improve alopecia, the present inventors have found that alkanamidoammonium compounds have an excellent hair generating effect as well as low skin irritation, and have accomplished the present invention based on this finding.

The present invention is directed to a hair generation promoting agent comprising as an effective ingredient an alkanamidoammonium compound represented by the formula:

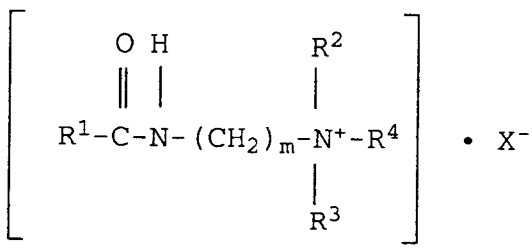

(I)

(wherein $R^1$ is an alkyl group having 7 to 25 carbon atoms; $R^2$, $R^3$ and $R^4$, which may be the same or different one another, are each an alkyl group having 1 to 4 carbon atoms; m is an integer of 2 to 4; and X is an atom or atomic group acting as an anion.

Furthermore, the present invention is directed to a method for promoting hair generation of a mammal comprising topically applying an effective amount of the alkanamidoammonium compound of Formula (I).

In addition, the present invention is directed to a use of the alkanamidoammonium compound of Formula (I) for the manufacture of a hair generation promoting agent.

In the present invention, the alkyl group having 7 to 25 carbon atoms refers to, for example, a heptyl group, an octyl group, a nonyl group, a decyl group, an undecyl group, a dodecyl group, a tetradecyl group, a pentadecyl group, a hexadecyl group, a heptadecyl group, an octadecyl group, a nonadecyl group, an icosyl group, a henicocyl group, a dococyl group, a tricocyl group and a tetracocyl group, and may be arranged as straight or branched chains.

The alkyl group having 1 to 4 carbon atoms refers to, for example, a methyl group, an ethyl group, a propyl group and a butyl group, and may be arranged as straight or branched chains.

Examples of the atom or atomic group acting as an anion are a fluorine atom, a chlorine atom, a bromine atom, an iodine atom, a sulfuric acid group, a nitric acid group, a nitrous acid group, a methyl sulfate group, an ethyl sulfate group, a dimethyl phosphate group, a methanesulfonic acid group, a benzenesulfonic acid group and a p-toluenesulfonic acid group.

Among the preferred compounds of Formula (I) are (coconut oil fatty acid amidopropyl)-trimethylammonium = chloride, [3-(decanamido)propyl]trimethylammonium = iodide, [2-(hexadecanamido)-ethyl]trimethylammonium = p-toluenesulfonate, [3-(docosanamido)propyl]trimethylammonium = iodide, [3-(tetradecanamido)propyl]dimethylethylammonium = bromide, [2-(octadecanamido)ethyl]-trimethylammonium = iodide, (tricosanamidopropyl)trimethylammonium = iodide, (octadecanamidoethyl)-

trimethylammonium = iodide, [2-(hexadecanamido)ethyl]dimethylethylammonium = iodide and [3-(octadecanamido)propyl]trimethylammonium = iodide. Most preferred among them are [3-(docasanamido) propyl] trimethylammonium = iodide.

As the effective ingredient of the present invention, one or more compounds of Formula (I) can be used. The effective ingredient can be topically applied in the dosage forms such as lotions, emulsions, creams, gels and earosols, all of which can be prepared by usual methods. The amount of the effective ingredient is 0.001 - 40% by weight, preferably 0.5 - 10% by weight based on the total formulation.

The preparation thus obtained can be applied to the scalp several times (preferably each once in the morning and night) a day in a suitable amount (preferably 0.5 - 2.0 ml).

Into the local preparation of the present invention can be incorporated, besides the above-mentioned effective ingredient, substances used for ordinary hair generation promoting agents, for example, solvents (e.g. ethyl alcohol, isopropyl alcohol, 1,3-butylene glycol, 1,4-butylene glycol, propylene glycol, dipropylene glycol, glycerol, diglycerol, polyethylene glycol and purified water), mucopolysaccharides (e.g. hyaluronic acid, condroitin-4-sulfate, condroitin-6-sulfate, dermatan sulfate, keratan sulfate, heparan sulfate and condroitin polysulfate), preservatives (e.g. parabens and benzoic acid), vitamins [e.g. vitamin A, vitamin $B_1$, vitamin $B_2$, vitamin $B_6$, vitamin C, vitamin D, vitamin E and derivatives thereof (e.g. vitamin E acetate)], oils (e.g. liquid paraffin, white soft paraffin, solid paraffin, ceresin, micro-crystalline wax, cholesterol, squalene, olive oil, rose hip oil, mink oil, jojoba oil, hydrogenated castor oil, hydrogenated coconut oil, isopropyl myristate, ethyl caproate, ethyl caprylate, palmitoleic acid, ethyl palmitolate, ethyl linolate, ethyl linolenoate, oleic acid, stearic acid, linoleic acid, linolenic acid, palmitic acid, behenic acid, lauric acid, stearyl alcohol, cetyl alcohol, lauryl alcohol, oleyl alcohol and cetyl isooctanoate), germicides [e.g. sulfur, chlorhexidine gluconate, chlorhexidine hydrochloride, cetyl pyridinium chloride, dequalinium chloride, isopropyl methyl phenol, quaternary ammonium salts (e.g. benzalkonium chloride) and hinokitiol], nonionic surfactants (e.g. polyoxy-ethylene fatty acid esters, polyoxyethylene sorbitan fatty acid esters, sorbitan fatty acid esters, glycerol fatty acid esters, polyoxyethylene glycerol fatty acid esters, polyglycerol fatty acid ester, propylene glycol fatty acid esters, polyglycerol alkyl phenyl ethers, polyoxyethylene hydrogenated castor oil and copolymers of polyoxyethylene and polypropylene glycol), anionic surfactants (e.g. N-acylamino acid salts, N-acylsarcosine salt, alkylphosphate salt and acylmethyltaurine salt), cationic surfactants (e.g. alkyltrimethyl ammonium, dialkyldimethylammonium and alkyl-N,N-dialkylaminoacetic acid esters), amphoteric surfactants [e.g. betaines (e.g. carboxybetaine and sulfobetaine), imidazoline and amineoxide], high-molecular surfactants (e.g. casein), silicone derivatives (e.g. silicone oil, polyol denatured silicone and silicone resin), thickeners (e.g. methylcellulose, polyvinylpyrrolidone, carboxymethylcellulose, carboxyethylcellulose, hydroxypropylcellulose and carboxyvinyl polymers), clay minerals (e.g. montmorillonite, saponite and hectolite), pH regulators (e.g. diisopropanolamine and citric acid), anti-oxidants (e.g. dibutylhydroxytoluene, potassium hydrogen sulfite, catechin and gluconodeltalactone), whitening agents (e.g. albutin and kojic acid), refrigerants (e.g. ℓ-menthol and camphor), anti-inframmatory agents (e.g. glycyrrhetic acid, dipotassium glycyrrhizinate, berberine chloride, shikonin, quaiazulene, allantoin and δ-aminocaproic acid), peripheral vasodilators (e.g. methyl nicotinate, benzyl nicotinate, swertiamarin, minoxidil, diasoxide, capsicum extract, capsicin and calpronium chloride), adrenocortical hormones (e.g. hydrocortisone acetate and betamethasone valerate), antihistamines (diphenhydramine hydrochloride and isothipendyl hydrochloride), local anesthetics (e.g. dibucaine hydrochloride and lidocaine hydrochloride), keratolytics (e.g. urea and salicylic acid), keratoregulators [e.g. vitamin A acid and derivative thereof (e.g. 13-cis-retinoic acid and etoretinate)], estrogens (e.g. 17β-estradiol, ethynylestradiol and estrone), progestins (e.g. progesterone and 17α-hydroxyprogesterone acetate), anti-androgens (e.g. cyproterone acetate and 4-androstene-3-one-17β-carboxylic acid), perfumes, sequestering agent, ultraviolet absorbents, moistening agents (e.g. snake gourd extract, ginseng extract, diisopropyl acetate, pyrrolidone carboxylic acid, polyglutamic acid, polyoxyalkylenealkylglycoside ether, collagen, lecithin, ceramide and 2-aminoethanesulfonic acid) and crude drug extracts (e.g. cashew extract, panax rhizome extract, lansic extract and saffran extract). The amounts of the substances are not so much as to degrade the effect of the present invention.

## BEST MODE OF CARRYING OUT THE INVENTION

The present invention is further illustrated in more detail by the following preparations of the compounds of Formula (I), examples and experiments.

(preparation)

[3-(Tetracosanamido)propyl]trimethylammonium = iodide

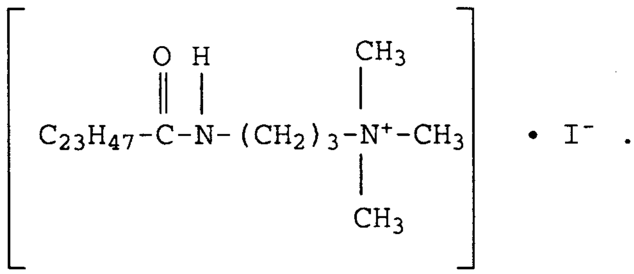

(1) Ten grams of tetracosan acid and 3.3 g of N,N-dimethyl-1,3-diaminopropane in xylene were refluxed for 5 hours, and the resulting product was recrystallized from xylene-n-hexane to give 11.8 g of N-[3-(dimethylamino)propyl] tetracosanamide.

(2) Two grams of the compound obtained above was reacted with 1.25 g of methyl iodide in ethanol at room temperature for 3 days, the resulting product was recrystallized from ethanol to give 1.77 g of the title compound.

m.p. 121 - 123 °C.

1H-NMR ($CDCl_3$)(δ ppm): 0.89(3H, m), 1.28(40H, s), 1.52-1.69(2H, m), 1.85-2.02(2H, m), 2.18(2H, t, J = 8Hz), 3.18-3.28(8H, m), 3.53-3.67(2H, m), 3.78(2H, s)

Other novel compounds among those of Formula (I) can be also prepared according to the foregoing preparation.

(Example 1)

| Ingredients | Amounts |
|---|---|
| (Coconut oil fatty acid amidopropyl)trimethylammonium = chloride, | 5 g |
| Dibutylhydroxytoluene | 0.1 g |
| Isopropylmethylphenol | 0.1 g |
| Vitamin $B_6$ | 0.5 g |
| Polyoxyethylene monostearate | 1.0 g |
| Methylcellulose | 0.2 g |
| Dipotassium glycyrrhizinate | 0.2 g |
| Capsicum tincture | 0.1 g |
| Propylene glycol | 5 g |
| Glycerol | 5 g |
| Purified water | Total 100 g |

The ingredients were mixed and dissolved in purified water, propylene glycol and glycerol to give a lotion.

(Example 2)

| Ingredients | Amounts |
|---|---|
| [3-(Decanamido)propyl]trimethylammonium = iodide | 10 g |
| 1,3-Butylene glycol | 5 g |
| Hydrocortisone acetate | 0.0016 g |
| Isothipendyl hydrochloride | 0.1 g |
| Sulfur | 3.0 g |
| Estrone | 0.0008 g |
| Ethyl alcohol | 50 g |
| Purified water | Total 100 g |

The ingredients were mixed and dissolved in purified water, ethyl alcohol and 1,3-butylene glycol to give a lotion.

(Example 3)

| Ingredients | Amounts |
|---|---|
| [2-(Hexadecanamido)ethyl]trimethylammonium = p-toluenesulfonate | 2 g |
| Propylene glycol | 3 g |
| Decyl-N,N-dimethyl-aminoacetate | 1 g |
| Polyoxyethylene hydrogenated castor oil | 3 g |
| Isopropyl alcohol | 5 g |
| Dipropylene glycol | 8 g |
| Purified water | Total 100 g |

The ingredients were mixed and dissolved in purified water, isopropyl alcohol, propylene glycol and dipropylene glycol to give a lotion.

(Example 4)

| Ingredients | Amounts |
|---|---|
| [3-(Docosanamido)propyl]trimethyl-ammonium=iodide | 2 g |

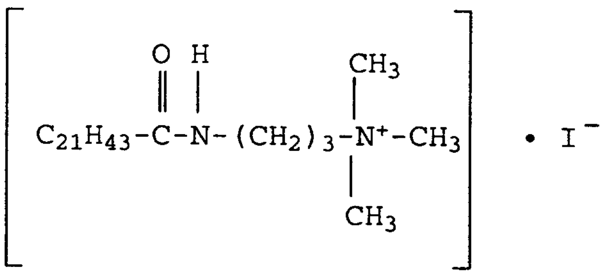

| | | |
|---|---|---|
| Ethyl alcohol | Total | 100 g |

[3-(Docosanamido)propyl]trimethylammonium = iodide was dissolved in ethyl alcohol to give a lotion.

(Example 5)

| Ingredients | Amounts |
|---|---|
| [3-(Tetradecanamido)propyl]dimethylethylammonium = bromide | 3 g |
| Vitamin E acetate | 0.2 g |
| Glycyrrhetic acid | 0.2 g |
| Glycerol monocaprate | 1 g |
| Squalene | 5 g |
| Liquid paraffin | 15 g |
| White soft paraffin | 3 g |
| Polyoxyethylene(20) sorbitan monostearate | 4 g |
| Purified water | Total 100 g |

The oil-soluble ingredients and the surfactant were dissolved with warming in an oily phase. Thereto were added a water phase obtained by dissolving [3-(tetradecanamido)propyl]-dimethylethylammonium = bromide and the water-soluble ingredients with warming for emulsification, and the mixture was cooled to give a cream.

(Example 6)

| Ingredients | Amounts |
|---|---|
| [2-(Octadecanamido)ethyl]trimethylammonium = iodide | 2 g |
| Triacetin | 5 g |
| Squalene | 5 g |
| Ethyl alcohol | Total 100 g |

The ingredients were mixed and dissolved in ethyl alcohol to give a lotion.

(Example 7)

| Ingredients | Amounts |
|---|---|
| (Tricosanamidopropyl)trimethylammonium = iodide | 4 g |
| Minoxidil | 0.5 g |
| Squalene | 20 g |
| Ethyl alcohol | Total 100 g |

The ingredients were mixed and dissolved in ethyl alcohol to give a lotion.

(Example 8)

| Ingredients | Amounts |
|---|---|
| (Octadecanamidoethyl)trimethylammonium = iodide | 8 g |
| Cholesterol | 0.5 g |
| Squalene | 3 g |
| Stearyl alcohol | 1 g |
| Polyoxyethylene hydrogenated caster oil | 5 g |
| Polyoxyethylene sorbitan monostearate | 2 g |
| Benzalkonium chloride | 0.1 g |
| Purified water | Total 100 g |

The oil-soluble ingredients and the surfactant were dissolved with warming in an oily phase. Thereto were added a water phase obtained by dissolving (octadecanamidoethyl]trimethylammonium = iodide and the water-soluble ingredients with warming for emulsification, and the mixture was cooled to give an emulsion.

(Example 9)

Base Liquid

| Ingredients | Amount |
|---|---|
| [2-(Hexadecanamido)ethyl]dimethylethylammonium = iodide | 3 g |
| Allantoin | 2 g |
| Squalene | 2 g |
| Stearyl alcohol | 0.2 g |
| Polyoxyethylene hydrogenated castor oil | 1 g |
| Polyoxyethylene sorbitan monostearate | 0.5 g |
| Parabens | 0.15 g |
| Propylene glycol | 5 g |
| Ethyl alcohol | 20 g |
| Purified water | Total 100 g |

Fillers

Dimethyl ether

The base liquid was prepared in a manner to that of the preparation of the emulsion in Example 8, and the following aerosol agent was prepared in a usual filling manner.

| Formulation | |
|---|---|
| Base liquid | 30% by weight |
| Filler | 70% by weight |

(Example 10)

| Ingredients | Amounts |
|---|---|
| [3-(Octadecanamido)propyl]trimethylammonium = iodide | 3 g |
| Polyvinylpyrrolidone | 3 g |
| Carboxyvinyl polymer | 0.5 g |
| Parabens | 0.15 g |
| Ethyl alcohol | 25 g |
| 1,3-Butylene glycol | 6 g |
| Purified water | Total 100 g |

The ingredients were dissolved in purified water, and then the thickeners were added to the solution for dissolution to give a gel.

(Example 11)

| Ingredients | | Amounts |
|---|---|---|
| [2-(Octadecanamido)ethyl]trimethyl-ammonium=iodide | | 2 g |
| 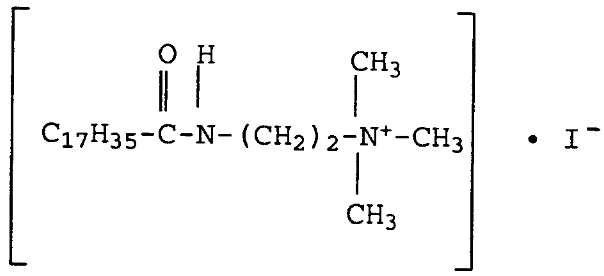 | | |
| Ethyl alcohol | Total | 100 g |

[2-(Octadecanamido)ethyl]trimethylammonium = iodide was dissolved in ethyl alcohol to give a lotion.

(Experiment) Test of the Hair Generation Promoting Effect

Groups each of 20 male C3H mice, 7 weeks old, were used for a test of the hair generation promoting effect of alkanamidoammonium compounds. Dorsal hairs of the animals were shaved by a clipper, and each of 0.2 ml of the samples indicated in Tables 1 or 2 was applied once a day on the shaved area for 10 days. The degree of hair generation was evaluated macroscopically by the following five grades.

0 : No hair generation was observed.
1 : A few vellus hairs grow.
2 : A few terminal hairs grow.
3 : Terminal hairs grow on 25% of the shaved area.
4 : Terminal hairs grow on 50% of the shaved area.

The group unapplied with the sample as control was evaluated in a similar manner. Results on 28 and 42 days after initiation of the application of the sample. were shown in Tables 1 and 2, respectively. Similarly, the mean values of the evaluation were shown in Tables 1 and 2.

Table 1

| Number of C3H mice in each values of evaluation / Sample | Value of evaluation | | | | | Mean value of evaluation (Point) |
|---|---|---|---|---|---|---|
| | 0 | 1 | 2 | 3 | 4 | |
| Lotion of Example 4 | 12 | 0 | 4 | 4 | 0 | 1.0 |
| Lotion of Example 11 | 12 | 5 | 3 | 0 | 0 | 0.6 |
| Ethanol | 20 | 0 | 0 | 0 | 0 | 0 |
| Purified water | 20 | 0 | 0 | 0 | 0 | 0 |
| No application | 20 | 0 | 0 | 0 | 0 | 0 |

Table 2

| Number of C3H mice in each values of evaluation / Sample | Value of evaluation | | | | | Mean value of evaluation (Point) |
|---|---|---|---|---|---|---|
| | 0 | 1 | 2 | 3 | 4 | |
| Lotion of Example 4 | 8 | 0 | 0 | 8 | 4 | 2.0 |
| Lotion of Example 11 | 8 | 0 | 8 | 4 | 0 | 1.4 |
| Ethanol | 18 | 2 | 0 | 0 | 0 | 0.1 |
| Purified water | 19 | 1 | 0 | 0 | 0 | 0.1 |
| No application | 19 | 1 | 0 | 0 | 0 | 0.1 |

The significant differences of the test results in Tables 1 and 2 were calculated according to the method of Wilcoxon.

From the results on 28 days after initiation of the application of the sample, there were obtained the significant differences at the $p \leqq 1\%$ value between the group applied with the lotion of Example 4 and the groups applied with the solvents or the group without any applications, and there were also obtained the significant differences at the $p \leqq 1\%$ value between the group applied with the lotion of Example 11 and the

groups applied with the solvents or the group without any applications.

From the results on 42 days after initiation of the application of the sample, there were obtained the significant differences at the $p \leqq 0.1\%$ value between the group applied with the lotion of Example 4 and the groups applied with the solvents or the group without any applications, and there were also obtained the significant differences at the $p \leqq 0.1\%$ value between the group applied with the lotion of Example 11 and the groups applied with the solvents or the group without any applications.

Industrial Utilization

According to the present invention, the compound of Formula (I), when topically applied to mammals, are enable to remarkably promote hair generation with low skin irritation.

**Claims**

1. A hair generation promoting agent comprising as an effective ingredient an alkanamidoammonium compound represented by the formula:

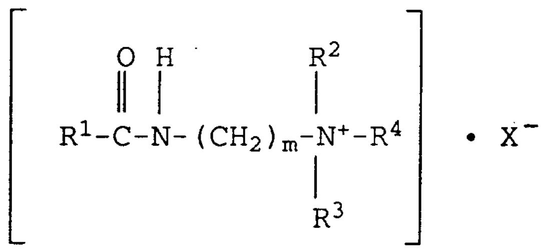

(wherein $R^1$ is an alkyl group having 7 to 25 carbon atoms; $R^2$, $R^3$ and $R^4$, which may be the same or different one another, are each an alkyl group having 1 to 4 carbon atoms; m is an integer of 2 to 4; and X is an atom or atomic group acting as an anion.

2. A hair generation promoting agent of Claim 1 wherein the alkanamidoammonium compound is [3-(docosanamido) propyl] trimethylammonium = iodide.

3. A method for promoting hair generation of a mammal comprising topically applying an effective amount of an alkanamidoammonium compound represented by the formula:

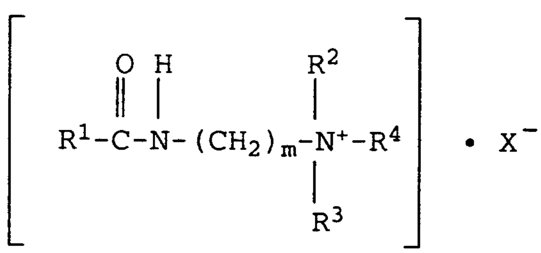

(wherein $R^1$ is an alkyl group having 7 to 25 carbon atoms; $R^2$, $R^3$ and $R^4$, which may be the same or different one another, are each an alkyl group having 1 to 4 carbon atoms; m is an integer of 2 to 4; and X is an atom or atomic group acting as an anion.

4. A method of Claim 3 wherein the alkanamidoammonium compound is [3-(docosanamido)propyl]-trimethylammonium = iodide.

5. A use of an alkanamidocarboxybetaine of an alkanamidoammonium compound represented by the formula: (wherein $R^1$ is an alkyl group having 7 to 25 carbon atoms;

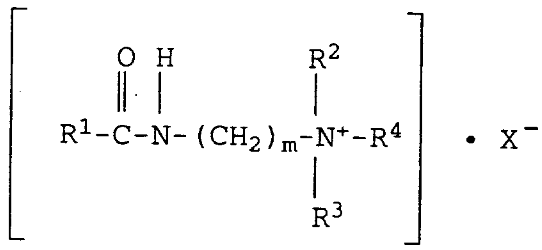

$R^2$, $R^3$ and $R^4$, which may be the same or different one another, are each an alkyl group having 1 to 4 carbon atoms; m is an integer of 2 to 4; and X is an atom or atomic group acting as an anion, for the manufacture of a pharmaceutical composition for a hair generation promoting agent.

**6.** A use of Claim 5 wherein the alkanamidoammonium compound is [3-(docosanamido)propyl]-trimethylammonium = iodide.

# INTERNATIONAL SEARCH REPORT

International Application No PCT/JP91/01416

**I. CLASSIFICATION OF SUBJECT MATTER** (if several classification symbols apply, indicate all) [6]

According to International Patent Classification (IPC) or to both National Classification and IPC

Int. $Cl^5$ A61K7/06

**II. FIELDS SEARCHED**

Minimum Documentation Searched [7]

| Classification System | Classification Symbols |
|---|---|
| IPC | A61K7/06 |

Documentation Searched other than Minimum Documentation to the Extent that such Documents are Included in the Fields Searched [8]

| | |
|---|---|
| Jitsuyo Shinan Koho | 1926 - 1991 |
| Kokai Jitsuyo Shinan Koho | 1971 - 1991 |

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 61-3778 (Ban Daic and Co., Inc.), February 4, 1986 (04. 02. 86) & BR, A, 8502591 & US, A, 4585692 & AU, B, 569951 & EP, B, 164688 & CA, A, 1264003 | 1-6 |
| X | JP, A, 62-192315 (Sunstar Corp.), August 22, 1987 (22. 08. 87), (Family: none) | 1-6 |
| Y | JP, A, 61-35968 (Ban Daic and Co., Inc.), August 15, 1986 (15. 08. 86), (Family: none) | 1-6 |
| Y | JP, A, 02-124811 (Sunstar Corp.), May 14, 1990 (14. 05. 90), (Family: none) | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 28, 1991 (28. 11. 91) | December 17, 1991 (17. 12. 91) |
| **International Searching Authority** | **Signature of Authorized Officer** |
| Japanese Patent Office | |